(19) Europäisches Patentamt
European Patent Office
Office européen des brevets

(11) Publication number: 0 125 827 A1

# (12) EUROPEAN PATENT APPLICATION

(21) Application number: 84302901.8

(22) Date of filing: 30.04.84

(51) Int. Cl.[3]: C 07 C 129/16
C 07 C 149/437, A 01 N 47/44
A 61 K 7/22

(30) Priority: 09.05.83 GB 8312662

(43) Date of publication of application: 21.11.84 Bulletin 84/47

(84) Designated Contracting States: AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Eakin, Murdoch Alan
2 Cockhall Lane
Langley Macclesfield Cheshire(GB)

(72) Inventor: Gunn, Donald Murray
16 Merlin Park
Clackmannan Scotland(GB)

(72) Inventor: Pemberton, Dennis
9 Eskdale Avenue
Bramhall Cheshire(GB)

(74) Representative: Atkinson, John David et al,
Imperial Chemical Industries PLC Legal Department : Patents PO Box 6 Bessemer Road
Welwyn garden City Herts, AL7 1HD(GB)

## (54) Polyether bisbiguanides.

(57) This invention relates to a polyether bisbiguanide derivative of the formula:-

$$R^1R^2N.C(:NR^7)NH.C(:NH)NR^3\text{-}X\text{-}NR^4.C(:NH)NH.C(:NR^8)NR^5R^6 \qquad \text{VIII}$$

or a tautomer thereof, wherein $R^1$, $R^2$, $R^5$ and $R^6$, which may be the same or different, are each hydrogen, a 1-16C alkyl radical, a 2-16C alkoxyalkyl radical, a 3-12C cycloalkyl radical, a (3-12C cycloalkyl)-(1-4C alkyl) radical, an optionally substituted phenyl, phenyl (1-4C alkyl) or phenyl (1-4C alkoxy) 1-4C alkyl) radical, or $R^1$ and $R^2$ and the nitrogen atom to which they are attached, or $R^5$ and $R^6$ and the nitrogen atom to which they are attached, which may be the same or different, are each a l-azetidinyl, 1-pyrrolidinyl, piperidino, hexamethyleneimino, heptamethyleneimino, morpholino or 4-(1-8C alkanoyl)-1-piperazinyl radical; each of which may bear 1-3C alkyl substituents; $R^3$ and $R^4$, which may be the same or different, are each hydrogen or 1-16C alkyl radical, a 3-16C cycloalkyl radical, a (3-12C cycloalkyl)-(1-4C alkyl) radical, an optionally substituted phenyl radical, an optionally substituted phenyl (1-4C alkyl) or naphthyl (1-4C alkyl) radical, or an optionally substituted diphenylmethyl radical; $R^7$ and $R^8$ are each hydrogen or a 1-8C alkyl radical; and X is a linking group of the formula:-

$$-(CH_2)_x-Z^1.Y.Z^2-(CH_2)_y- \qquad \text{IX}$$

wherein $Z^1$ and $Z^2$ are each an oxygen or sulphur atom, Y is a polymethylene, *p*-phenylene, 1,4-phenylene-dimethylene or 1,5-naphthylene radical, a radical of the formula:-

$$-C(CH_3)_2- \qquad \text{X}$$

or a polymethylene radical which is interrupted by up to 5 ether oxygen or thioether sulphur atoms, and x and y are integers being 2 or greater, such that the total number of methylene groups in the linking group X is not more than 18; and the acid-addition salts thereof; to processes for their manufacture; and to compositions and methods of use thereof.

EP 0 125 827 A1

POLYETHER BISBIGUANIDES

This invention relates to bisbiguanide derivatives, and in particular to polyether bisbiguanides which possess antibacterial properties.

Certain bisbiguanides are well-known as antibacterial, particularly antiseptic, agents. For example, in United Kingdom patent specification No. 705,838 there are disclosed and claimed bisbiguanides of the general formula:-

$$A-NH.C(:NH)NH.C(:NH)NH-(CH_2)_n-NH.C(:NH)NH.C(:NH)NH-A \qquad I$$

wherein A stands for a phenyl radical which is substituted by alkyl, alkoxy, nitro or halogen, and wherein the two A's may be the same or different and wherein n is an integer from 3 to 9 inclusive, and wherein the polymethylene chain may be interrupted by oxygen atoms and/or by aromatic nuclei. The compounds are said to be useful as bactericides; for example, those of the formula:-

$$A'-NH.C(:NH)NH.C(:NH)NH-(CH_2)_{5-7}-NH.C(:NH)NH.C(:NH)NH.A' \qquad II$$

wherein A' stands for a halogen substituted phenyl radical, possess very high antibacterial activity when tested *in vitro* against the organisms *Steptococcus haemolyticus*, *Staphylococcus aureus*, *Bacillus coli*, *Clostridium welchii* and *Pseudomonas pyocyanea*.

Similarly, in United Kingdom patent specification No. 1,095,902 there is disclosed and claimed a broad group of bisguanides and bisbiguanides which includes _inter alia_ compounds of the formula:-

$$RR'N-[-C(:NH)NH-]_x-A^2-[NH.C(:NH)-]_x-NRR' \qquad III$$

wherein $A^2$ stands for an alkylene radical of 2 to 12 carbon atoms having the valency bonds attached to different carbon atoms, or for a group of the formula:-

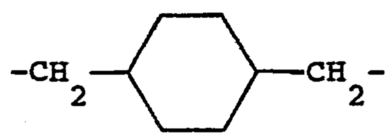

IV

R stands for an alkyl radical of 6 to 16 carbon atoms, R' stands for hydrogen, and x stands for 1 or 2. The bisguanides and bisbiguanides are said to have particular usefulness as plant fungicides and bactericides.

None of these disclosures, however, particularly describes any bisbiguanide compound wherein the group linking the two biguanide residues is a polyether.

A paper by Rose and Swain, J. Chem. Soc., 1956, 4422-4425, describes _inter alia_ three bisbiguanides in which the linking group is an ether, namely:-

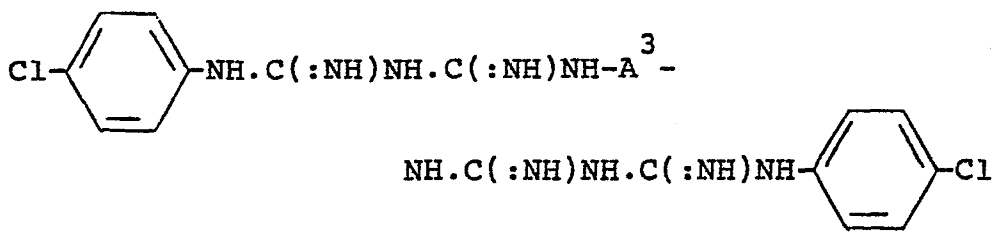

$A^3 = (CH_2)_3O(CH_2)_3$ V

$(CH_2)_3O(CH_2)_2O(CH_2)_3$ VI

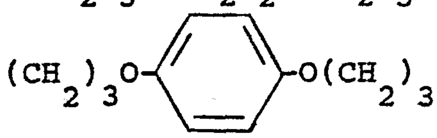 VII

but none of these compounds is mentioned as being of particular interest as an antibacterial agent in the paper by Rose and Swain's co-workers, Davies _et al_., Brit. J. Pharmacol., 1954, _9_, 192, which describes antibacterial results for compounds of interest made by Rose and Swain.

A series of patents from the Procter and Gamble Company, United Kingdom Patents Numbers 1,432,487, 1,449,302 and 1,507,846 and United States Patent Number 4,025,616, relate to specialised toothpaste, mouthwash, etc. compositions containing certain bisbiguanides, including _inter alia_ such compounds in which the two biguanide residues are linked by a 2-12C polymethylene chain "which can be interrupted by up to 5 ether, thioether, phenyl or naphthyl moities". The synthesis of bisbiguanides containing such linking groups is not described, but it is alleged that "the bis-biguanide compounds of this invention are known, having been disclosed in U.S. Patent 2,684,924, Rose et al., U.S. Patent 2,990,425, Senior et al., U.S. Patent 2,830,006, Burtwell (sic) et al., and U.S. Patent 2,863,019, Burtwell (sic) et al." (This latter number is an error for 2,863,919, which is shown correctly in United Kingdom Patent No. 1,507,846). Birtwell and Senior were co-workers of Rose, and the corresponding United Kingdom

Patents are respectively Numbers 705,838 (Rose), 815,925 (Senior), 745,064 (Birtwell) and 785,937 (Birtwell). However, the allegation that the Procter and Gamble bisbiguanides are known from the patents of Rose, Senior and Birtwell is completely incorrect insofar as it purports to relate to bisbiguanides in which the biguanide residues are linked by a polyether. The only polyether linking group specifically described by Rose, Senior or Birtwell is that shown in formula VI above.

United Kingdom Patent Number 1,401,518 discloses bisbiguanides wherein the two biguanide groups are joined by a monoether linking group, for use as anti-acne agents.

Thus, according to the present invention, there is provided a polyether bisbiguanide derivative of the formula:-

$$R^1R^2N.C(:NR^7)NH.C(:NH)NR^3-X-NR^4.C(:NH)NH.C(:NR^8)NR^5R^6$$

VIII

or a tautomer thereof, wherein $R^1$, $R^2$, $R^5$ and $R^6$, which may be the same or different, are each hydrogen, a 1-16C alkyl radical, a 2-16C alkoxyalkyl radical, a 3-12C cycloalkyl radical, a (3-12C cycloalkyl)-(1-4C alkyl) radical, an optionally substituted phenyl, phenyl(1-4C alkyl) or phenyl(1-4C alkoxy)(1-4C alkyl) radical, or $R^1$ and $R^2$ and the nitrogen atom to which they are attached, or $R^5$ and $R^6$ and the nitrogen atom to which they are attached, which may be the same or different, are each a 1-azetidinyl, 1-pyrrolidinyl, piperidino, hexamethyleneimino, heptamethyleneimino, morpholino or 4-(1-8C alkanoyl)-1-piperazinyl radical; each of which may bear 1-3C alkyl substituents; $R^3$ and $R^4$, which may be the

same or different, are each hydrogen or 1-16C alkyl radical, a 3-16C cycloalkyl radical, a (3-12C cycloalkyl)-(1-4C alkyl) radical, an optionally substituted phenyl radical, an optionally substituted phenyl(1-4C alkyl) or naphthyl(1-4C alkyl) radical, or an optionally substituted diphenylmethyl radical; $R^7$ and $R^8$ are each hydrogen or a 1-8C alkyl radical; and X is a linking group of the formula:-

$$-(CH_2)_x-Z^1.Y.Z^2-(CH_2)_y- \qquad IX$$

wherein $Z^1$ and $Z^2$ are each an oxygen or sulphur atom, Y is a polymethylene, p-phenylene, 1,4-phenylenedimethylene or 1,5-naphthylene radical, a radical of the formula:-

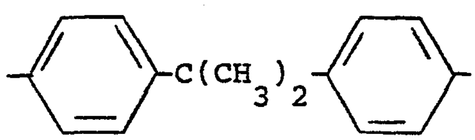

X

or a polymethylene radical which is interrupted by up to 5 ether oxygen or thioether sulphur atoms, and x and y are integers being 2 or greater, such that the total number of methylene groups in the linking group X is not more than 18; and the acid-addition salts thereof.

Each of $R^1$, $R^2$, $R^3$, $R^4$, $R^5$ and $R^6$ may be, for example, a methyl, ethyl, n-propyl, isopropyl, n-butyl, isobutyl, sec-butyl, tert-butyl, pentyl, neopentyl, octyl, 2-ethylhexyl, dodecyl, hexadecyl, cyclobutyl, cyclopentyl, cyclohexyl, cycloheptyl, cyclopentylmethyl or cyclohexylmethyl radical, particularly the ethylhexyl, n-hexyl and cyclohexyl radicals.

Each of $R^7$ and $R^8$ may be, for example, a methyl, ethyl, n-propyl, isopropyl, n-butyl, isobutyl, sec-butyl, tert-butyl, pentyl, neopentyl or octyl radical.

When any of $R^1$, $R^2$, $R^3$, $R^4$, $R^5$ and $R^6$ is an optionally substituted phenylalkyl radical, it is preferably a benzyl, α-methylbenzyl, α-ethylbenzyl or phenethyl radical, and suitable optional substituents in the phenyl ring thereof or in $R^1$, $R^2$, $R^3$, $R^4$, $R^5$ and $R^6$ when any of them is a phenyl radical are, for example, halogen atoms, for example chlorine, bromine, iodine or fluorine atoms, amino, carbamoyl, cyano, hydroxy, nitro and trifluoromethyl radicals, 1-6C alkyl, alkoxy, alkanoyl, alkylamino and alkanoylamino radicals and 2-6C alkoxycarbonyl and dialkylamino radicals. Suitable such radicals are, for example, methyl, ethyl, propyl, butyl, pentyl, hexyl, methoxy, ethoxy, propoxy, acetamido, propionamido, butyramido, methylamino, ethylamino, propylamino, acetyl, propionyl, butyryl, methoxycarbonyl, ethoxycarbonyl, dimethylamino and diethylamino radicals. Up to five such substituents may be present, but mono- and di- substituted phenyl rings are preferred, and especially mono-substituted rings.

Thus, further suitable values for $R^1$, $R^2$, $R^3$, $R^4$, $R^5$ and $R^6$ are, for example, 2-, 3- and 4-chlorophenyl, 2-, 3- and 4-bromophenyl, 2-, 3- and 4-flurophenyl, 2,3-, 2,4-, 2,5-, 2,6-, 3,4- and 3,5-dichlorophenyl, 2-chloro-4-fluorophenyl, 2-, 3- and 4-methylphenyl, 2,3-, 2,4-, 2,5-, 2,6-, 3,4- and 3,5-dimethylphenyl, 2-, 3- and 4-methoxyphenyl, 2-, 3- and 4-acetylphenyl, 2-, 3- and 4-methylaminophenyl, 2-, 3- and 4-acetamidophenyl, 2-, 3- and 4-methoxycarbonylphenyl, 2-, 3- and 4-dimethylaminophenyl, 2-, 3- and 4-nitrophenyl,

2-, 3- and 4-chlorobenzyl, 2-, 3- and 4-bromobenzyl, 2-, 3- and 4-fluorobenzyl, 2,3-, 2,4-, 2,5-, 2,6-, 3,4- and 3,5-dichlorobenzyl, 2-chloro-4-fluorobenzyl, 2-, 3- and 4-methylbenzyl, 2,3-, 2,4-, 2,5-, 2,6-, 3,4- and 3,5-dimethylbenzyl, 2-, 3- and 4-methoxybenzyl, 2-, 3- and 4-acetylbenzyl, 2-, 3- and 4-methylaminobenzyl, 2-, 3- and 4-acetamidobenzyl, 2-, 3- and 4-methoxycarbonylbenzyl, 2-, 3- and 4-dimethylaminobenzyl, 2-, 3- and 4-nitrobenzyl, 2-, 3- and 4-chloro-α-methylbenzyl, 2-, 3- and 4-nitrobenzyl, 2-, 3-and 4-chlorophenethyl and bis-(2-,3- and 4-chlorophenyl)methyl radicals.

When one or more of $R^1$, $R^2$, $R^5$ and $R^6$ is an alkoxyalkyl radical, it may be, for example, a 2-methoxyethyl, 3-methoxypropyl, 3-hexyloxypropyl, 6-hexyloxyhexyl, 2-tetradecyloxyethyl or 15-methoxypentadecyl radical.

When $R^1$ and $R^2$, and $R^5$ and $R^6$, together with the nitrogen atom to which they are attached, form a heterocyclic radical, preferred such radicals are the 1-pyrrolidinyl and piperidino radicals.

The term "polymethylene" in the definition of Y above is to be understood to include, for example, methylene, ethylene, trimethylene and hexamethylene. Thus, particular values for X are, for example, the following:-

$-(CH_2)_2O(CH_2)_2O(CH_2)_2-$ XI

$-(CH_2)_3O(CH_2)_2O(CH_2)_3-$ XII

$-(CH_2)_3O(CH_2)_4O(CH_2)_3-$ XIII

$-(CH_2)_3O(CH_2)_8O(CH_2)_3-$ XIV

$-(CH_2)_3O(CH_2)_2O(CH_2)_2O(CH_2)_3-$ XV

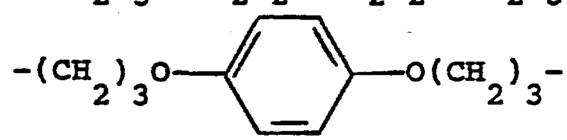

XVI

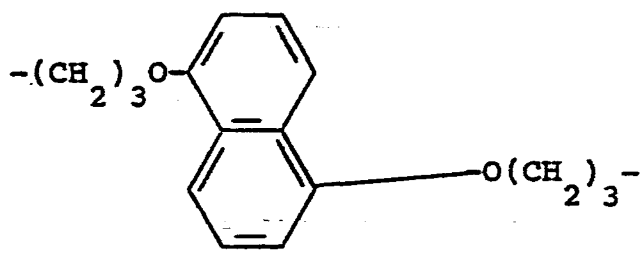

XVII

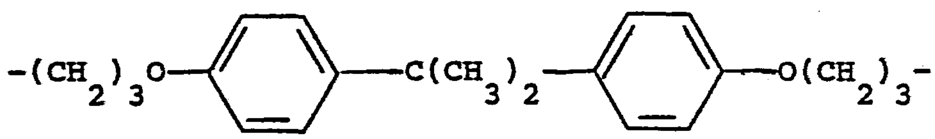

XVIII

The acid-additon salts of the invention may be derived from an inorganic or organic acid. In most circumstances it is preferable that the salts be derived from an acid which affords an anion which is suitable for human usage, for example a pharmaceutically-acceptable anion. Examples of such acids are hydrochoric, hydrobromic, phosphoric, sulphuric, acetic, D-gluconic, 2-pyrrolidone-5-carboxylic acids, methanesulphonic, carbonic, lactic and glutamic acids.

Particular preferred polyether bisbiguanides of the invention are 4,7-dioxadecanebis[5-(2-ethylhexyl)-biguanide], 4,9-dioxadodecanebis[5-(2-ethylhexyl)-biguanide], 4,9-dioxadodecanebis(1-benzyl-5-cyclohexyl-biguanide), O,O'-bis[3-(5-hexylbiguanido)propyl]hydroquinone, 4,9-dioxadodecanebis[1-(2,4-dichlorobenzyl-5-ethylbiguanide] and 3,6-dioxa-octanebis[5-(2-ethylhexyl)-biguanide], and their dihydrochlorides.

According to a further feature of the invention there is provided a process for the manufacture of the compounds of the invention wherein $R^7$ and $R^8$ are each hydrogen, which comprises reacting a bis-cyanoguanidine of the formula:-

$$NC.NH.C(:NH)NR^3-X-NR^4.C(:NH).NH.CN \quad XIX$$

with an amine, $R^1R^2NH$, or successively with two different amines, $R^1R^2NH$ and $R^5R^6NH$ in the form of an acid acid-addition salt thereof, wherein X, $R^1$, $R^2$, $R^3$, $R^4$, $R^5$ and $R^6$ have the meanings stated above, at a temperature of $100^o$ to $170^oC$.

A suitable amine salt is, for example, the hydrochloride. The reactants are heated together until the reaction is complete. The reaction proceeds fastest at higher temperatures, but if thermal stability is a problem, the reaction is carried out at lower temperature over a longer period. The reactants are most conveniently melted together in the absence of a solvent, but if desired an inert solvent such as 2-methoxyethanol, 2-ethoxyethanol, nitrobenzene, sulpholane, isopropanol, n-butanol, ethylene glycol dimethyl ether or water, or a mixture of such solvents, may be used.

This bis-cyanoguanidine of the formula XIX, which may be used as the starting material in the above process, may be obtained by reacting the appropriate diamine $NHR^3-X-NHR^4$, in the form of an acid-addition salt, conveniently the dihydrochloride, with sodium dicyanamide, in conventional manner.

According to a further feature of the invention there is provided a process for the manufacture of the compounds of the invention which comprises reacting a diamine of the formula $R^3NH-X-NHR^4$, in the form of an acid-addition salt, with a cyanoguanidine of the formula:-

$$R^1R^2N.C(:NR^7)NH.CN \qquad XX$$

or successively with a cyanoguanidine of the formula XX and a cyanoguanidine of the formula:-

$$R^5R^6N.C(:NR^8)NH.CN \qquad XXI$$

and wherein X, $R^1$, $R^2$, $R^3$, $R^4$, $R^5$, $R^6$, $R^7$ and $R^8$ have the meanings stated above, at a temperature of $100^o$ to $170^oC$.

A suitable salt of the diamine is, for example, the dihydrochloride. The reactants are heated together until the reaction is complete. The reaction proceeds fastest at higher temperatures, but where thermal stability is a problem, the reaction is carried out at lower temperature for a longer period. If a melt can be formed at those temperatures the reactants are conveniently melted together in the absence of a solvent. If not, or alternatively, the reactants are heated together in a suitable inert solvent, for example those mentioned above.

The amine of the formula $R^3NH-X-NHR^4$, which is used as the starting material in the above process, may be obtained by alkylation of the primary diamine $NH_2-X-NH_2$ in conventional manner, for example by reaction with an alkyl halide, or by reductive alkylation with an aldehyde.

The cyanoguanidines of the formulae XX and XXI, wherein $R^7$ and $R^8$ are hydrogen, which are used as the starting materials in the above process, may be obtained by reacting sodium dicyanamide with an appropriate amine $R^1R^2NH$ or $R^5R^6NH$, in the form of an acid-addition salt, conveniently the dihydrochloride, in a suitable inert solvent.

The cyanoguanidines of the formulae XX and XXI wherein $R^7$ and $R^8$ are is other than hydrogen, which may be used as starting materials in the above process, may be obtained by reacting a dialkyl (cyanoimido)-dithiocarbonate, for example dimethyl (cyanoimido)-dithiocarbonate, $(MeS)_2C:N.CN$, with appropriate amines $R^1R^2NH$ and $R^7NH_2$ (which are preferably the same), or $R^5R^6NH$ and $R^8NH_2$.

The acid-addition salts of the invention are obtained by conventional means.

The antibacterial activity of the compounds of the invention has been measured by the well-known minimum inhibitory concentration (MIC) test. Neat or diluted broth cultures of eight Gram positive organisms (Streptococcus pyogenes, S. faecalis, 3 strains of Staphyloccus aureus, Listeria monocytogenes, Streptococcus mutans, S. sanguis), Candida albicans and fourteen Gram negative organisms (4 strains of Escherichia coli, Salmonella dublin, Klebsiella aerogenes, K. pneumoniae, E. cloacae, Serratia marcescens, Proteus vulgaris, P. mirabilis and 3 strains of Pseudomonas aeruginosa) were inocculated by means of an automatic mirotitre innoculator on the surface of nutrient agar plates containing two-fold or five fold dilutions of a test compound. After incubation overnight at 37°C., the MIC's of the test compound are read. The

geometric mean MIC's for the Gram positive organisms and Candida, and 14 Gram negative organisms are then calculated for each test compound.

Depending upon its precise chemical structure, a compound of the invention has a mean MIC within the range 1-12 μg./ml. in agar against the 8 Gram positive organisms and Candida, and 20-250 μg./ml. in agar against the 14 Gram negative organisms.

The preferred compounds of the invention have an acute $LD_{50}$ within the accepted limits for compounds used topically, are of low irritancy in the Draize test on intact rabbit skin, are negative in the Ames test for mutagenicity, and are non-sensitizing in the Magnusson and Kligman contact sensitivity test in guinea-pigs.

Because of their antibacterial and/or antifungal properties, the compounds of the invention are useful for many purposes, for example:-

(a) in medical and veterinary practice for the disinfection of wounds, membranes and/or skin tissue: and

(b) for the sterilisation of surgical instruments and other medical apparatus and equipment, for example respirators, ventilators, incubators, humidifiers, etc.;

(c) for incorporation in toothpastes and mouthwashes for inhibiting the formation of dental plaque, and gingivitis;

(d) for the disinfection of hard surfaces, for example plant and equipment used in the food and drink industries, and floors and walls in the home, factories and hospitals;

(e) for the disinfection of textiles, for example blankets, overalls, bed-linen, etc.;

(f) for the control of microbiological slime in the pulp and paper industries;

(g) for the control of micro-organisms in swimming pools, cooling water, pasteuriser water, aqueous oil emulsions such as metal working fluids, and other circulating water systems; and

(h) as plant bactericides and fungicides.

Compounds of the invention also possess useful antifungal activity, for example, Candida albicans or Trichophyton mentagrophytes, and algicidal and anti-yeast activity.

According to a further feature of the invention there are provided antibacterial or antifungal compositions comprising a compound of the formula VIII wherein $R^1$, $R^2$, $R^3$, $R^4$, $R^5$, $R^6$, $R^7$, $R^8$ and X have the meanings stated above, or an acid-addition salt therof, and an inert diluent or carrier therefor.

The antibacterial or antifungal compositions of the invention are prepared by conventional means using conventional excipients. They include, for example, aqueous solutions, for example concentrated aqueous solutions, sterile, ready-to-use aqueous solutions,

aqueous dispersions, and emulsions, for example oil-in-water emulsions, for example aqueous gels, creams, ointments and pastes. Suitable excipients include, for example, wetting agents, dispersing agents, emulsifying agents, gelling agents or thickening agents.

According to a further feature of the invention, there is provided a contraceptive method which comprises applying to sperm or the locus of sperm, a spermicidal, sperm-immobilising or mucospissic amount of a compound of the invention of the formula VIII.

In one aspect of this method, the compound of the formula VIII, when applied to the vaginal mucus at suitable concentration, very rapidly increases its viscosity, to the extent that it becomes essentially impenetrable to sperm, and forms a physical barrier to conception in the same way as a rubber sheath or a diaphragm cap.

Besides increasing the viscosity of vaginal mucus, when the mucus comes into contact with a bisbiguanide compound of the formula VIII, other changes occur in its intrinsic properties, such as its morphology, rheology and water uptake and visco-elastic properties, which can also effect its penetrability to sperm. The compounds also possess spermicidal or sperm-immobilising properties.

_In vitro_, the compounds of the formula VIII exert a useful contraceptive effect at concentrations down to about $10^{-3}$ to $10^{-4}$%, and a suitable amount to be applied to the human vagina for contraceptive purposes is from 1.0g. to $10^{-4}$g.

The compound of the formula VIII may be applied to the vagina in conventional manner, for example as a pessary, cream, liquid douche, gel, aerosol foam or impregnated tampon, or in a controlled delivery device of the compound in a polymer matrix.

According to a further feature of the invention there is provided a bisbiguanide compound of the formula VIII, or a composition thereof, for use as a contraceptive.

The invention is illustrated but not limited by the following Examples in which the temperatures are expressed in degrees Celsius. Medium pressure liquid chromatography (MPLC) was on Merck Lichoprep RP-18, or C18 column eluting with aqueous methanol. Fractions containing bisbiguanide were detected by UV absorption at 236 nm., $\epsilon \geqslant 25000$ for products in which each biguanide group bears one substituent in addition to the linking group X, or 245 nm, $\epsilon \geqslant 32000$ for products bearing two such substituents.

Example 1

An intimate mixture of 1,4-phenylenebis(2-methylthioethylamine) dihydrochloride (1.1 g.) and 3-cyano-1-isopropylguanidine (1.26 g.) was heated at 160° (bath temperature) for 2 hours. The cooled reaction mixture was dissolved in methanol (10 ml.) and the solution was filtered. The filtrate was added to acetone (150 ml.) and the crystalline precipitate was collected to give 4,4'-p-phenylenebis[3-thiabutyl(5-isopropylbiguanide)] dihydrochloride m.p. 172-174°.

The 1,4-phenylenebis(2-methylthioethylamine) dihydrochloride used as starting material for the above example may be prepared as follows:-

1,4-bis-(Bromomethyl)benzene (10.6 g.) was added to a stirred mixture of 2-aminoethanethiol hydrochloride (9.5 g.), ethanol (70 ml.) and a solution of sodium hydroxide (6.8 g.) in water (30 ml.), and the mixture was stirred at room temperature for 18 hours. The mixture was evaporated to dryness and the residue was partitioned between 1N hydrochloric acid and diethyl ether. The aqueous phase was basified with 10N sodium hydroxide solution and then extracted twice with ethyl acetate. The combined ethyl acetate extracts were dried and then evaporated to dryness. The residue was dissolved in acetonitrile and the solution was acidified with a solution of hydrogen chloride in diethyl ether, and the crystalline precipitate was collected to give 4,4'-p-phenylenebis(3-thiabutylamine) dihydrochloride m.p. 290° (decomposition),

<u>Examples 2 - 50</u>

The process described in Example 1 was repeated, using the appropriate diamine and the appropriate cyanoguanidine as starting materials, to manufacture the following compounds:-

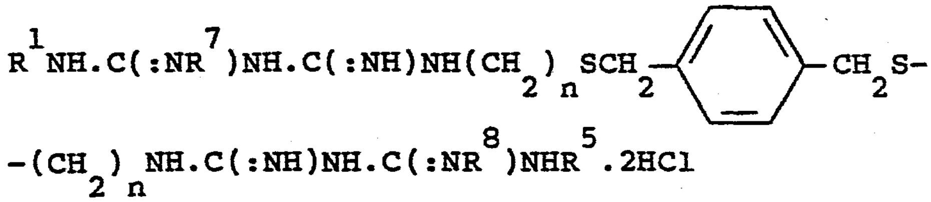

| Ex | $R^1 (=R^5)$ | $R^7 (=R^8)$ | n | Reaction time (hours) | Crystal-lisation solvent | m.p. |
|---|---|---|---|---|---|---|
| 2 | methyl | H | 2 | 1.5 | a | 131-134 |
| 3 | propyl | H | 2 | 2 | a | 164-166 |
| 4 | butyl | H | 2 | 2 | b | 169-172 |
| 5 | hexyl | H | 2 | 3 | c | 129-131xz |
| 6 | cyclohexyl | H | 2 | 2 | a | 204-207 |
| 7 | benzyl | H | 2 | 2 | a | 155-157 |
| 8 | phenethyl | H | 2 | 2 | gum | |
| 9 | methyl | methyl | 2 | 1.5 | a | 203-205 |
| 10 | ethyl | ethyl | 2 | 2 | a | 220-221 |
| 11 | propyl | propyl | 2 | 3 | d | 185-187 |
| 12 | butyl | H | 3 | 3 | c | 178-179xz |
| 13 | cyclohexyl | H | 3 | 3 | a | 167-170x |

$$R^1R^2N.C(:NH)NH.C(:NH)NR^3.(CH_2)_3O(CH_2)_4O(CH_2)_3-$$
$$-NR^4.C(:NH)NH.C(:NH)NR^5R^6.2HCl$$

| Ex | $R^1$ (= $R^5$) | $R^2$ (= $R^6$) | $R^3$ (= $R^4$) | Reaction time (hours) | Crystal-lisation solvent | m.p. |
|---|---|---|---|---|---|---|
| 14 | isopropyl | H | benzyl | 2.5 | c | 174-175xz |
| 15 | benzyl | H | benzyl | 2.5 | c | 132-133xz |
| 16 | cyclohexyl | H | benzyl | 2.5 | b | 160-161x |
| 17 | H | H | 2,4-$Cl_2$-benzyl | 2 | c | 106-108xz |
| 18 | methyl | methyl | 2,4-$Cl_2$-benzyl | 3 | c | 204-206xz |
| 19 | ethyl | H | 2,4-$Cl_2$-benzyl | 2 | b | 167-169x |
| 20 | propyl | H | 2,4-$Cl_2$-benzyl | 2 | c | 181-183xz |
| 21 | H | H | 3,4-$Cl_2$-benzyl | 2.5 | c | 142-144xz |
| 22 | methyl | H | 3,4-$Cl_2$-benzyl | 2.5 | e | 169-171x |
| 23 | ethyl | H | 3,4-$Cl_2$-benzyl | 2.5 | c | 180-182xz |
| 24 | isopropyl | H | 3,4-$Cl_2$-benzyl | 2.5 | e | 189-191x |
| 25 | methyl | methyl | 3,4-$Cl_2$-benzyl | 2.5 | c | 199-200xz |

$R^1NH.C(:NH)NH.C(:NH)NR^3(CH_2)_3O$ 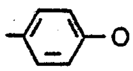 $O(CH_2)_3NR^4.-$
$-C(:NH)NH.C(:NH)NHR^5.2HCl$

| Ex | $R^1 (=R^5)$ | $R^3 (=R^4)$ | Reaction time (hours) | Crystal-lisation solvent | m.p. |
|---|---|---|---|---|---|
| 26 | methyl | H | 3 | f | 236-239 |
| 27 | butyl | H | 3 | g | 207-209 |
| 28 | cyclohexyl | H | 3 | g | 233-235 |
| 29 | benzyl | H | 3 | g | 218-220 |
| 30 | phenethyl | H | 3 | g | 197-199 |
| 31 | hexyl | H | 4 | c | 183-184xz |
| 32 | 2-ethylhexyl | H | 4 | c | 187-189xz |
| 33 | butyl | benzyl | 5 | c | 203-205yz |
| 34 | cyclohexyl | benzyl | 5 | a | 210-214y |
| 35 | hexyl | benzyl | 5 | c | 195-196yz |

$R^1R^2N.C(:NH)NH.C(:NH)NR^3(CH_2)_3O$ 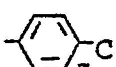 $C(CH_3)_2-$
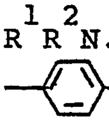 $O(CH_2)_3NR^4.C(:NH)NH.C(:NH)NR^5R^6.2HCl$

| Ex | $R^1$ (= $R^5$) | $R^2$ (= $R^6$) | $R^3$ (= $R^4$) | Reaction time (hours) | Crystal-lisation solvent | m.p. |
|---|---|---|---|---|---|---|
| 36 | ethyl | H | H | 3 | e | 153-156x |
| 37 | propyl | H | H | 3 | h | 151-153x |
| 38 | isopropyl | H | H | 3 | e | 196-199x |
| 39 | isobutyl | H | H | 3 | e | 165-170x |
| 40 | hexyl | H | H | 3 | c | 178-180xz |
| 41 | cyclohexyl | H | H | 3 | e | 210-213x |
| 42 | H | H | benzyl | 2.5 | c | 211-214xz |
| 43 | methyl | H | benzyl | 2.5 | e | 181-184x |
| 44 | ethyl | H | benzyl | 2.5 | c | 203-205xz |
| 45 | propyl | H | benzyl | 2.5 | c | 154-157xz |
| 46 | methyl | methyl | benzyl | 2.5 | c | 145-150xz |

$R^1NH.C(:NH)NH.C(:NH)NH(CH_3)_3O$

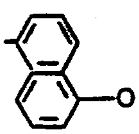

$-O(CH_2)_3NH.C(:NH)-$

$-NH.C(:NH)NHR^5.2HCl$

| Ex | $R^1$ (= $R^5$) | Reaction time (hours) | Crystal-lisation solvent | m.p. |
|---|---|---|---|---|
| 47 | hexyl | 2 | c | 162-165yz |
| 48 | cyclohexyl | 2 | c | 251-253yz |

$R^1R^2N.C(:NR^7)NH.C(:NH)NH(CH_2)_3O(CH_2)_2O(CH_2)_3$-
$NH.C(:NH)NHC(:NR^8)NR^5R^6.2HCl$

| Ex | $R^1(=R^5)$ | $R^2(=R^6)$ | $R^7(=R^8)$ | Reaction time (hours) | m.p. |
|---|---|---|---|---|---|
| 49 | isopentyl | isopentyl | H | 3 | gum |
| 50 | isopentyl | H | isopentyl | 3 | gum |

Compounds in Examples 8, 49 and 50, described as gums, were adequately characterised by nuclear magnetic resonance spectroscopy.

a - methanol/acetone
b - ethanol/acetonitrile
c - acetone
d - methanol/acetonitrile
e - ethanol/acetone
f - ethanol
g - water
h - acetonitrile
x - reaction mixture diluted with a small volume of sulpholane prior to heating
y - reaction mixture diluted with a small volume of dimethylsulphoxide prior to heating
z - purified by MPLC

The 1,4-phenylenebis(3-methylthiopropylamine), used as starting material in Examples 12 and 13, was manufactured as follows:-

3-Bromopropylamine hydrobromide (7.9 g.) was added to a mixture of p-xylene-$\alpha,\alpha'$-dithiol (2.55 g.), ethanol (40 ml.) and 10N sodium hydroxide solution (10 ml.), and the mixture was heated at reflux for 18 hours. The mixture was evaporated to dryness, and the residue was partitioned between 2N aqueous hydrochloric acid and diethyl ether. The aqueous phase was separated, basified with 10N sodium hydroxide solution and extracted with ethyl acetate, and the ethyl acetate extract was dried and evaporated to dryness. A solution of the residue in acetonitrile was acidified with a solution of hydrogen chloride in diethyl ether, and the precipitated solid was collected and recrystallised from ethanol to give the required diamine which was used without further purification.

The 4,9-dioxadodecane-1,12-di(benzylamine) dihydrochloride used as starting material for Examples 14-16 was manufactured as follows:-

A solution of 4,9-dioxadodecane-1,12-diamine (6.12 g.) and benzaldehyde (5.7 g.) in ethanol (100 ml.) was mixed with 5% palladium on carbon, and the mixture was shaken under hydrogen at room temperature and atmospheric pressure until the uptake of hydrogen had ceased. The catalyst was filtered off, and the filtrate was acidified to pH 1 with concentrated hydrochloric acid, and then evaporated to dryness. The residue was crystallised from ethanol to give 4,9-dioxadodecane-1,12-di(benzylamine) dihydrochloride, m.p. 191-194°.

In a similar manner, starting from the appropriate diamines, there were obtained:

1,4-bis(3-benzylaminopropoxy)benzene dihydrochloride, starting material for Examples 33-35, m.p. 292-295° from aqueous ethanol; and

2,2-bis[4-(3-benzylaminopropoxy)phenyl]propanedihydrochloride, starting material for Examples 42-46, m.p. 227-230°, from methanol/acetonitrile.

The 4,9-dioxadodecane-1,12-di(2,4-dichlorobenzylamine)dihydrochloride used as starting material for Examples 17-20 was manufactured as follows:-

A mixture of 4,9-dioxadodecan-1,12-diamine diacetate (3.24 g.), 2,4-dichlorobenzaldehyde (3.85 g.), ethanol (100 ml.) and platinum IV oxide (0.3 g.) was shaken under hydrogen at room temperature and atmospheric pressure until 450 mls. of hydrogen had been absorbed. The mixture was filtered and the filtrate was evaporated to dryness. The residue was stirred with a mixture of 2N hydrochloric acid (30 ml.) and ether, and the insoluble solid was collected and recrystallised twice from ethanol to give 4,9-dioxadodecane-1,12-di(2,4-dichlorobenzyl-amine) dihydrochloride m.p. 196-198°.

The 4,9-dioxadodecane-1,12-di(3,4-dichlorobenzylamine) dihydrochloride used as starting material for Examples 21-25 was manufactured as follows:-

A mixture of 4,9-dioxadodecane-1,12-diamine (3.88 g.), potassium carbonate (5.5 g.), ethanol (20 ml.) and 3,4-dichlorobenzyl chloride (7.82 g.) was stirred at room temperature for 72 hours. The mixture was filtered

and the filtrate was evaporated to dryness. The residue was stirred with a mixture of ether and 2N aqueous hydrochloric acid, and the insoluble solid collected, and recrystallised twice from ethanol, to give 4,9-dioxadodecane-1,12-di(3,4-dichlorobenzylamine) dihydrochloride m.p. 253-255°.

The 1,5-bis(3-aminopropoxy)naphthalene dihydrochloride used as starting material for Examples 47 and 48 was manufactured as follows:-

A mixture of 1,5-dihydroxynaphthalene (16 g.), acrylonitrile (21.2 g.), a 40% aqueous solution of triton B (N-benzyltrimethylammonium hydroxide) (3 ml.) and acetonitrile (200 ml.) was refluxed for 30 hours and then filtered. The filtrate was evaporated to dryness and the residue was stirred with 0.5N aqueous sodium hydroxide. The insoluble material was collected to give 1,5-bis(2-cyanoethoxy)naphthalene which was used without further purification.

A solution of 1,5-bis(2-cyanoethoxy)naphthalene (1.0 g.) in ethanolic ammonia was treated with Raney Nickel, and the mixture shaken under hydrogen at room temperature and atmospheric pressure until the uptake of hydrogen ceased. The catalyst was filtered off and the filtrate was evaporated to dryness. The residue was dissolved in ethanol, the solution was acidifed with concentrated hydrochloric acid, and the precipitated solid was collected to give 1,5-bis(3-aminopropoxy)-naphthalene dihydrochloride m.p. $>$300°.

The 3-cyano-1-hexylguanidine used as starting material for Examples 5, 35, 40 and 47 was manufactured as follows:-

A mixture of hexylamine hydrochloride (63.5 g.), sodium dicyanamide (44.5 g.) and butanol (200 ml.) was heated at reflux for 18 hours and then cooled. The mixture was filtered and the filtrate was evaporated to dryness. The residue was stirred with water and the insoluble solid was collected and crystallised from aqueous ethanol, to give 3-cyano-1-hexylguanidine, m.p. 103-105°.

Example 51

A mixture of 4,9-dioxadodecane 1,12-di(isobutylamine)dihydrochloride (0.53 g.), n-butanol (2 ml.) and 3-cyano-1,1-di-isobutylguanidine (1.18 g.) was stirred at 150¯ (bath temperature) in an open-necked flask for 5 hrs. The cooled reaction mixture was triturated with acetone, and the insoluble solid was collcted and recrystallised from a mixture of ethanol and acetonitrile to give 4,9-dioxadodecane-1,12-bis(1,5,5-tri-isobutylbiguanide) dihydrochloride, m.p. 199-201°.

The 3-cyano-1,1-di-isobutylguanidine used as starting material in the above process was prepared by the process described for the manufacture of 3-cyano-1-hexylguanidine at the end of Examples 2-50; m.p. 100-101°, after crystallisation from toluene-petroleum ether (b.p. 60-80°).

Example 52

The process describd in Example 51 was repeated using 4,9-dioxadodecane-1,12-di(benzylamine) dihydrochloride and 3-cyano-1,1-dipropylguanidine as starting materials, adding a small volume of sulpholane to the initial reaction mixture, and heating for 3 hours, to

give 4,9-dioxadodecane-1,12-bis(1-benzyl-5,5-dipropylbiguanide) dihydrochloride, m.p. 214-216° after crystallisation from acetone.

The 3-cyano-1,1-dipropylguanidine used as starting material in the above process, was manufactured as follows:-

n-Propylamine (40 g.) was added to a solution of dimethyl (cyanoimido)dithiocarbonate (20 g.) in ethanol (100 ml.) with stirring. Sufficient N,N-dimethylformamide was added to redissolve the white solid which precipitated, and the solution was kept at room temperature for 24 hours. The solution was evaporated to dryness, the residue was triturated with ethyl acetate, and the insoluble solid was collected to give 3-cyano-1,1-dipropylguanidine which was used without further purification.

Example 53

The process described in Example 52 was repeated using 4,9-dioxadodecane-1,12-di(benzylamine) dihydrochloride and 1,1-dibutyl-3-cyanoguanidine as starting materials, to give 4,9-dioxadodecane-1,12-bis(1-benzyl-5,5-dibutylbiguanide) dihydrochloride, m.p. 213-215° after purification by MPLC.

Example 54

A mixture of 4,9-dioxododecane-1,12-di(isobutylamine) dihydrochloride (0.975 g.), 1,1-dibutyl-3-cyanoguanidine (1.96 g.) and n-butanol (5 ml.) was stirred at reflux for 48 hours and then evaporated to dryness. The residue was triturated with acetone and the

insoluble solid collected. This crude product was purified by MPLC, and the fractions containing the required bisbiguanide were combined and evaporated to dryness. The residue was triturated with acetone and the insoluble crystalline solid was collcted to give 4,9-dioxadodecane-1,12-bis(5,5-dibutyl-1-isobutylbiguanide) dihydrochloride m.p. 209-211°.

The 4,9-dioxadodecane-1,12-di(isobutylamine) dihydrochloride used in Examples 14-16 was prepared as follows:-

A solution of 4,9-dioxadodecane-1,12-diamine (6.12 g.) and isobutyraldehyde (4.6 g.) in ethanol (100 ml.) was treated with 5% palladium on carbon catalyst and the mixture shaken under hydrogen at room temperature and atmospheric pressure until the uptake of hydrogen had ceased. The catalyst was filtered off and the filtrate was acidified to pH1 with concentrated hydrochloric acid and then evaporated to dryness. The residue was crystallised from isopropanol to give 4,9-dioxadodecane-1,12-di(isobutylamine) dihydrochloride m.p. 235-237°

Example 55

An intimate mixture of 1,4-bis[3-(3-cyanoguanidino)propoxy]benzene (0.716 g.) and propylamine hydrochloride (1.52 g.) was heated in a bath at 150° for 2.5 hours. The crude reaction mixture was dissolved in isopropanol (10 ml.) and the solution was diluted with acetone (200 ml.). The solid that precipitated was collected and recrystallised from isopropanol to give 1,4-bis[3-(5-propylbiguanido)propoxy]benzene dihydrochloride, m.p. 183-186°.

Example 56

The process described in Example 55 was repeated, using decylamine hydrochloride in place of propylamine hydrochloride, to give 1,4-bis[3-(5-decylbiguanido)propoxy]benzene dihydrochloride, m.p. 158-161°.

Examples 57-77

The appropriate diamine and the appropriate substituted cyanoguanidine were fused together at 150° for 3 hours, and the cooled product was purified by MPLC to give the following products:-

$R^1R^2N.C(:NH)NH.C(:NH)NR^3.(CH_2)_3O(CH_2)_4O(CH_2)_3-$
$-NH.C(:NH)NH.C(:NH)NR^5R^6.2HCl$

| Ex | $R^1$ (=$R^5$) | $R^2$ (=$R^6$) | $R^3$ | m.p. |
|---|---|---|---|---|
| 57 | neopentyl | H | H | 175-176 |
| 58 | hexyl | H | H | 132-136 |
| 59 | 1-methyl-hexyl | H | H | 194-197 |
| 60 | octyl | H | H | 150-154 |
| 61 | 2-ethyl-hexyl | H | H | 172-174 |
| 62 | 1,5-dimethyl-hexyl | H | H | 191-193 |
| 63 | isobutyl | isobutyl | H | gum |
| 64 | $-CH_2CH_2OCH_2CH_2-$ | | dodecyl | gum |
| 65 | methyl | H | dodecyl | gum |

$R^1R^2N.C(:NH)NH.C(:NH)NH(CH_2)_3O(CH_2)_4O-$
$-(CH_2)_3NH.C(:NH)NH.C(:NH)NR^5R^6.2HCl$

| Ex | $R^1$ | $R^2$ | $R^5$ | $R^6$ | m.p. |
|---|---|---|---|---|---|
| 66 | dodecyl | methyl | methyl | methyl | gum |
| 67 | dodecyl | H | methyl | methyl | gum |
| 68 | dodecyl | H | ethyl | H | 117-120 |

$R^1R^2N.C(:NR^7)NH.C(:NH)NH(CH_2)_m(OCH_2CH_2)_n-$
$-O(CH_2)_mNH.C(:NH)NH.C(:NR^8)NR^5R^6.2HCl$

| Ex | $R^1$ | $R^2$ | $R^5$ | $R^6$ | $R^7$ (=$R^8$) | m | n |
|---|---|---|---|---|---|---|---|
| 69 | 2-ethyl-hexyl | H | 2-ethyl-hexyl | H | H | 3 | 2a |
| 70 | isopentyl | isopentyl | isopentyl | isopentyl | H | 3 | 2 |
| 71 | isopentyl | H | isopentyl | H | iso-pentyl | 3 | 2 |
| 72 | heptyl | H | heptyl | H | H | 2 | 1 |
| 73 | 2-ethyl-hexyl | H | 2-ethyl-hexyl | H | H | 2 | 1 |
| 74 | 1-methyl-hexyl | H | 1-methyl-hexyl | H | H | 3 | 1 |
| 75 | 2-ethyl-hexyl | H | 2-ethyl-hexyl | H | H | 3 | 1 |

a - m.p. 159-162°

Example 76

A mixture of 4,7,10-trioxatridecane-1,13-diamine dihydrochloride (4.0 g.), 3-cyano-1-tridecylguanidine (3.64 g.) and 3-cyano-1-methylguanidine (1.34 g.) was fused at 150° for 3 hours. The reaction mixture from this mixed fusion, containing three bisbiguanide products, was purified by MPLC, and the eluate fractions containing the major product (identified by thin layer chromatography), were combined and evaporated to dryness under reduced pressure, to give 13-(5-methylbiguanido)-4,7,10-trioxatridecane-1-(5-tetradecylbiguanide), as a gum. The structure and purity of the product were confirmed by microanalysis, U.V. spectrum, and fast atom bombardment mass spectrometry, (FAB-MS).

Examples 77-87

The process described in Example 78 was repeated, using the appropriate diamine dihydrochloride and cyanoguanidines as starting materials, to produce the following analogous compounds.

$$R^1R^2N.C(:NR^7)NH.C(:NH)NH(CH_2)_m(OCH_2CH_2)_nO(CH_2)_mNH.C-$$
$$-(:NH)NH.C(:NR^8)NR^5R^6.2HCl$$

| Ex | $R^1$ | $R^2$ | $R^3$ | $R^4$ | $R^5$ | m | n |
|---|---|---|---|---|---|---|---|
| 77 | dodecyl | H | methyl | H | H | 3 | 2 |
| 78 | tetradecyl | H | H | H | H | 3 | 2 |
| 79 | tetradecyl | H | 2-benzyl-oxyethyl | H | H | 3 | 2 |
| 80 | dodecyl | H | methyl | methyl | H | 3 | 2 |
| 81 | tridecyl | H | methyl | H | H | 3 | 2 |
| 82 | dodecyl | H | $-CH_2CH_2OCH_2CH_2-$ | | H | 3 | 2 |
| 83 | dodecyl | H | isopropyl | H | H | 2 | 1 |
| 84 | isobutyl | isopentyl | isopentyl | isopentyl | H | 3 | 1 |
| 85 | dodecyl | H | methyl | H | H | 3 | 1 |
| 86 | dodecyl | H | methyl | methyl | H | 3 | 1 |
| 87 | tetradecyl | H | methyl | methyl | H | 3 | 1 |

The 4,7,10,13,16,19,22-heptaoxapentacosane-1,25-diamine, used as starting material for the manufacture of the compounds of Examples 72 and 73, may be obtained as follows:-

3,6,9,12,15-pentaoxaheptadecane-1,17-diol (25.2 g.) and sodium hydride (20 mg. as 50% suspension in oil) were stirred at 25° for 30 minutes. Acrylonitrile (9.47 g.) was added dropwise to this stirred mixture and the reaction temperature was maintained at below 30° by cooling the reaction vessel in a water bath. The reaction mixture was stirred overnight at room temperature, then toluene (50 ml.) was added and the mixture was reduced to small volume by evaporation under reduced pressure. The product was purified by chromatography on silica gel, using acetone:methylene dichloride (1:1 by volume) as eluant, to give 3,6,9,12,15,18,21-heptaoxatricosyl-1,23-dinitrile as a

yellow oil ($R_f$ = 0.5 on thin layer chromatography using silica plates, acetone/methylene chloride (3:7 by volume) as the developing solvent and iodine as the visualizing agent).

The above product (23 g.) was dissolved in a solution of liquid ammonia (20 ml.) in ethanol (180 ml.). Catalyst (3.3 g., 5% rhodium on alumina) was added, and the mixture was hydrogenated under pressure (50 atmospheres) at 50° for 19 hours.

The mixture was filtered through celite, and the filtrate was diluted with toluene (200 ml.) then evaporated to small volume under reduced pressure, to give the required product, 4,7,10,13,16,19,22-heptaoxa-pentacosane-1,25-diamine as a pale brown oil ($R_f$ = 0.15 on silica tlc plate developed in ammonia/methanol (1:9 by volume) and visualised with iodine), which was converted to the dihydrochloride in conventional manner, and then used without further purification.

Example 88

A mixture of 4,7,10,13,16,19,22-heptaoxapenta-cosane-1,25-bis(3-cyanoguanidine) (2.65 g.) and nonylamine hydrochloride (2.65 g.) was fused at 145° for 3.5 hours. The fusion mixture was cooled and purified by MPLC to give 4,7,10,13,16,19,22-heptaoxapentacosane-1,25-bis(5-nonylbiguanide) as a gum. The structure and purity of the product were confirmed by microanalysis, U.V. spectrum and fast atom bombardment mass spectometry.

The 4,7,10,13,16,19,22-heptaoxapentacosane-1,25-bis(3-cyanoguanidine) used as starting material in the above process was manufactured as follows:-

A solution of 4,7,10,13,16,19,22-heptaoxapentacosane-1,25-diamine, (obtained as described in the latter part of Examples 77-87) (17 g.) and sodium dicyandiamide (7.1 g.) in butanol (350 ml.), was heated under reflux for 4 hours. The reaction mixture was allowed to cool then filtered through celite. The filtrate was evaporated to small volume under reduced pressure and the crude product, obtained as an oil, was chromatographed on silica gel with methylene dichloride/acetone (1:1 by volume) as the eluant. The product, 4,7,10,13,16,19,22-heptaoxapentacosane-bis(3-cyanoguanidine), was obtained as an oil on evaporation of fractions containing the material with $R_f$ = 0.5 on silica thin layer chromatography developed in acetone and visualized with iodine. The product was characterised by fast atom bombardment mass spectroscopy.

Example 89

The process described in Example 88 was repeated, using decylamine hydrochloride in place of nonylamine hydrochloride, to give 4,7,10,13,16,19,22-heptaoxapentacosane-1,25-bis(5-decylbiguanide), also as a gum, and similarly characterised.

What we claim is:-

1. A polyether bisbiguanide derivative of the formula:-

$$R^1R^2N.C(:NR^7)NH.C(:NH)NR^3-X-NR^4.C(:NH)NH.C(:NR^8)NR^5R^6 \qquad VIII$$

or a tautomer thereof, wherein $R^1$, $R^2$, $R^5$ and $R^6$, which may be the same or different, are each hydrogen, a 1-16C alkyl radical, a 2-16C alkoxyalkyl radical, a 3-12C cycloalkyl radical, a (3-12C cycloalkyl)-(1-4C alkyl) radical, an optionally substituted phenyl, phenyl(1-4C alkyl) or phenyl(1-4C alkoxy)(1-4C alkyl) radical, or $R^1$ and $R^2$ and the nitrogen atom to which they are attached, or $R^5$ and $R^6$ and the nitrogen atom to which they are attached, which may be the same or different, are each a 1-azetidinyl, 1-pyrrolidinyl, piperidino, hexamethyleneimino, heptamethyleneimino, morpholino or 4-(1-8C alkanoyl)-1-piperazinyl radical; each of which may bear 1-3C alkyl substituents; $R^3$ and $R^4$, which may be the same or different, are each hydrogen or 1-16C alkyl radical, a 3-16C cycloalkyl radical, a (3-12C cycloalkyl)-(1-4C alkyl) radical, an optionally substituted phenyl radical, an optionally substituted phenyl(1-4C alkyl) or naphthyl(1-4C alkyl) radical, or an optionally substituted diphenylmethyl radical; $R^7$ and $R^8$ are each hydrogen or a 1-8C alkyl radical; and X is a linking group of the formula:-

$$-(CH_2)_x-Z^1.Y.Z^2-(CH_2)_y- \qquad IX$$

wherein $Z^1$ and $Z^2$ are each an oxygen or sulphur atom, Y is a polymethylene, p-phenylene, 1,4-phenylenedimethylene or 1,5-naphthylene radical, a radical of the formula:-

$-C(CH_3)_2-$ X

or a polymethylene radical which is interrupted by up to 5 ether oxygen or thioether sulphur atoms, and x and y are integers being 2 or greater, such that the total number of methylene groups in the linking group X is not more than 18; and the acid-addition salts thereof.

2. A compound as claimed in claim 1 wherein each of $R^1$, $R^2$, $R^3$, $R^4$, $R^5$ and $R^6$, which may be the same or different, is hydrogen or a methyl, ethyl, n-propyl, isopropyl, n-butyl, isobutyl, sec-butyl, tert-butyl, pentyl, neopentyl, octyl, 2-ethylhexyl, dodecyl, hexadecyl, cyclobutyl, cyclopentyl, cyclohexyl, cycloheptyl, cyclopentylmethyl, cyclohexylmethyl, or a phenyl, benzyl, $\alpha$-methylbenzyl, $\alpha$-ethylbenzyl or phenethyl radical each optionally substituted in the phenyl ring thereof by chlorine, bromine, iodine or fluorine atoms, or by amino, carbamoyl, cyano, hydroxy, nitro, trifluoromethyl, methyl, ethyl, propyl, butyl, pentyl, hexyl, methoxy, ethoxy, propoxy, acetamido, propionamido, butyramido, methylamino, ethylamino, propylamino, acetyl, propionyl, butyryl, methoxycarbonyl, dimethylamino or diethylamino radicals; or each of $R^1$, $R^2$, $R^5$ and $R^6$ which may be the same or different, is a 2-methoxyethyl, 3-methoxypropyl, 3-hexyloxypropyl, 6-hexyloxyhexyl, 2-tetradecyloxyethyl or 15-methoxypentadecyl radical; or $R^1$ and $R^2$, or $R^5$ and $R^6$, together with the nitrogen atom to which they are attached, form a 1-pyrrolidinyl or piperidino radical; or each of $R^3$ and $R^4$ is a naphthyl(1-4C)alkyl or diphenylmethyl radical optionally radical optionally substituted in a phenyl ring or rings thereof as defined above; each of $R^7$ and $R^8$ which may be the same or different, is hydrogen or a methyl, ethyl, n-propyl,

isopropyl, n-butyl, isobutyl, sec-butyl, tert-butyl, pentyl, neopentyl or octyl radical; and X is a diradical of the formula:-

$-(CH_2)_2O(CH_2)_2O(CH_2)_2-$ XI

$-(CH_2)_3O(CH_2)_2O(CH_2)_3-$ XII

$-(CH_2)_3O(CH_2)_4O(CH_2)_3-$ XIII

$-(CH_2)_3O(CH_2)_8O(CH_2)_3-$ XIV

$-(CH_2)_3O(CH_2)_2O(CH_2)_2O(CH_2)_3-$ XV

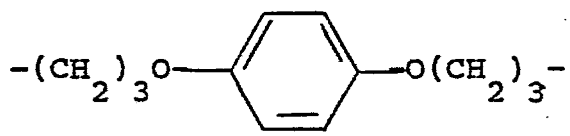

XVI

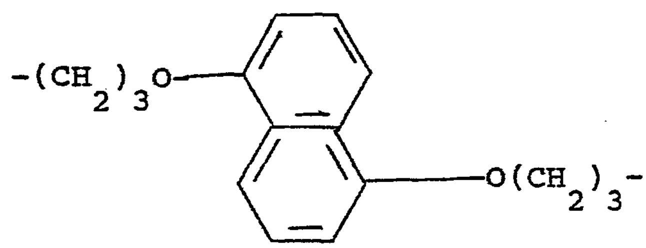

XVII

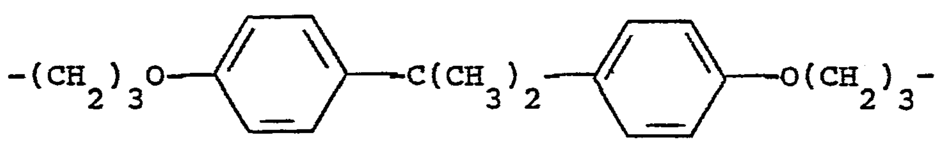

XVIII

3. A compound as claimed in claim 2 wherein each of $R^1$, $R^2$, $R^3$, $R^4$, $R^5$ and $R^6$, which may be the same or different, is a phenyl, benzyl, -methylbenzyl, -ethylbenzyl, phenethyl, 2-, 3- or 4-chlorophenyl, 2-, 3- or 4-bromophenyl, 2-, 3- or 4-fluorophenyl, 2,3-, 2,4-, 2,5-, 2,6-, 3,4- or 3,5-dichlorophenyl, 2-chloro-3-fluorophenyl, 2-, 3- or 4-methylphenyl, 2,3-, 2,4-, 2,5-, 2,6-, 3,4- or 3,5-dimethylphenyl, 2-, 3- or 4-methoxyphenyl, 2-, 3- or 4-acetylphenyl, 2-, 3- or 4-methylaminophenyl, 2-, 3- or

4-acetamidophenyl, 2-, 3- or 4-methoxycarbonylphenyl, 2-, 3- or 4-dimethylaminophenyl, 2-, 3- or 4-nitrophenyl, 2-, 3- or 4-chlorobenzyl, 2-, 3- or 4-bromobenzyl, 2-, 3- or 4-fluorobenzyl, 2,3-, 2,4-, 2,5-, 2,6-, 3,4- or 3,5-dichlorobenzyl, 2-chloro-4-fluorobenzyl, 2-, 3- or 4-methylbenzyl, 2,3-, 2,4-, 2,5-, 2,6-, 3,4- or 3,5-dimethylbenzyl, 2-, 3- or 4-methoxybenzyl, 2-, 3- or 4-acetylbenzyl, 2-, 3- or 4-methylaminobenzyl, 2-, 3- or 4-acetamidobenzyl, 2-, 3- or 4-methoxycarbonylbenzyl, 2-, 3- or 4-dimethylaminobenzyl, 2-, 3- or 4-nitrobenzyl, 2-, 3- or 4-chloro-  -methylbenzyl, 2-, 3- or 4-chlorophenethyl or bis(2-, 3- or 4-chlorophenyl)methyl radical; and X is a trimethylene, hexamethylene, undecamethylene, dodecamethylene or methylenbis(4-cyclohexyl) radical.

4. A compound as claimed in claim 1 which is in the form of a salt with hydrochloric, hydrobromic, phosphoric, sulphuric, acetic, D-gluconic, 2-pyrrolidone-5-carboxylic, methanesulphonic, carbonic, lactic or glutamic acid.

5. A compound as claimed in claim 1 which is 4,7-dioxadecanebis[5-(2-ethylhexyl)- biguanide], 4,9-dioxadodecanebis[5-(2-ethylhexyl)-biguanide], O,O'-bis[3-(5-hexylbiguanido)propyl]hydro- quinone, 4,9-dioxadodecanebis[1-(2,4-dichlorobenzyl-5-ethylbiguanide] and 3,6-dioxa-octanebis[5-(2-ethylhexyl)-biguanide], or a dihydrochloride thereof.

6. A process for the manufacture of a compound as claimed in claim 1 which comprises:-

(a) for those compounds wherein $R^7$ and $R^8$ are each hydrogen, reacting a bis-cyanoguanidine of the formula:-

$$NC.NH.C(:NH)NR^3\text{-}X\text{-}NR^4.C(:NH).NH.CN \qquad XIX$$

with an amine, $R^1$ $R^2NH$, or successively with two different amines, $R^1R^2NH$ or $R^5R^6NH$ in the form of an acid-addition salt thereof, wherein X, $R^1$, $R^2$, $R^3$, $R^4$, $R^5$ and $R^6$ have the meanings stated in claim 1, at a temperature of 100° to 170°C.; or

(b) reacting a diamine of the formula $R^3NH\text{-}X\text{-}NHR^4$, in the form of an acid addition salt, with a cyanoguanidine of the formula:-

$$R^1R^2N.C(:NR^7)NH.CN \qquad XX$$

or successively with a cyanoguanidine of the formula XX and a cyanoguanidine of the formula:-

$$R^5R^6N.C(:NR^8)NH.CN \qquad XXI$$

and wherein X, $R^1$, $R^2$, $R^3$, $R^4$, $R^5$, $R^6$, $R^7$ and $R^8$ have the meanings stated in claim 1, at a temperature of 100° to 170°C.

7. An antibacterial or antifungal composition comprising a compound as claimed in claim 1 and an inert diluent or carier therefore.

8. A method of obtaining an antibacterial or antifungal effect:

(a) in medicinal and veterinary practice for the disinfection of wounds, membranes or skin tissue;

(b) in the sterilisation of surgical instruments and other medical apparatus and equipment;

(c) in toothpastes and mouthwashes for inhibiting gingivitis and the formation of dental plaque;

(d) in the disinfection of hard surfaces;

(e) in the disinfection of textiles;

(f) in the control of microbiological slime in the pulp and paper industries;

(g) in the control of micro-organisms in swimming pools, cooling water, pasteuriser water, aqueous oil emulsions and other circulating water systems; and

(h) against plant bacteria and/or fungi;

which comprises applying an antibacterially- or antifungally-effective amount of a bisbiguanide as claimed in claim 1 to the bacterially or fungally affected locus.

9. A contraceptive method which comprises applying to sperm, or the locus of sperm, a spermicidal, sperm-immobilising or mucospissic amount of a compound as claimed in claim 1.

JOHN DAV... ..KINSON

DS32716
SC11 - 16 Apr 84
JDA/LMS

AUTHORISED REPRESENTATIVE
General Authorisation No. 97

European Patent Office

# EUROPEAN SEARCH REPORT

0125827
Application number

EP 84 30 2901

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | GB-A-1 095 902 (STERLING DRUG INC.) * Claims * ----- | 1-9 | C 07 C 129/16<br>C 07 C 149/437<br>A 01 N 47/44<br>A 61 K 7/22 |
| | | | TECHNICAL FIELDS SEARCHED (Int. Cl. 3) |
| | | | C 07 C 129/00 |

The present search report has been drawn up for all claims

| Place of search | Date of completion of the search | Examiner |
|---|---|---|
| THE HAGUE | 17-08-1984 | GAUTIER R.H.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82